# EUROPEAN PATENT APPLICATION

(11) **EP 1 911 460 A1**
(43) Date of publication of application: **16.04.2008**
(21) Application number: 07253973.7
(22) Date of filing: 08.10.2007
(51) Int. Cl.: A61K 38/51, C12N 9/88, A61P 25/00

(54) **Pharmaceutical composition for promoting axonal re-growth and behaviour recovery in spinal cord injury**

(30) Priority: 06.10.2006 US 828450 P
(71) Applicant: Cheng, Henrich, Taipei, Taiwan 112 (CN)
(72) Inventor: Cheng, Henrich, Taipei Taiwan 112 (CN); Huang, Wen-Cheng, Taipei Taiwan 112 (CN); Kuo, Wen-Chun, Taipei Taiwan 112 (CN)
(74) Representative: Wright, Simon Mark

(57) **Abstract**

A pharmaceutical composition for promoting axon re-growth and behaviour recovery in a subject suffering from a nerve injury is disclosed comprising a safe and effective amount of a chondroitinase ABC (ChABC), namely 0.1 to 10U/ml. This can be administered to an injury site of the nerve injury in the subject.

## Description

### BACKGROUND OF THE INVENTION

The present invention relates to a method and a pharmaceutical composition for promoting axonal re-growth and behaviour recovery in spinal cord injury.

In mammals, the adult neural tissues have limited regenerative capability. Therefore, numerous brain or spinal cord injuries are unable to repair themselves, or re-establish functional connections. Many complicated causes, including immunoresponse- molecules and inhibitory factors interfere with the regenerative capacity of adult central nervous system. Chondroitin sulphate proteoglycans (CSPGs) are up-regulated via astrocytes and oligodendrocytes in the injury site after spinal cord injury (SCI) to limit axonal regeneration.

Some reports suggest that CSPG expression is generally up-regulated after injuries, and most studies have focused on change in expression of single family members after different lesion methods. CSPGs are composed of glycosaminoglycan (GAG), which may be a key limiting factor to block axon growth. Chondroitinase ABC (ChABC) is a bacterial enzyme, which digests the GAG side chain of CSPGs. However, ChABC is rarely used in clinical trials of spinal cord injury cases due to its toxicity.

### BRIEF SUMMARY OF THE INVENTION

The present invention provides (the use of) an (e.g. bacterial) enzyme that can digest the GAG side chain of CSPGs, for example chondroitinase or ChABC (e.g. at a low dose) for treating nerve injury in a subject.

One aspect of the invention provides a method for promoting axon re-growth and behaviour recovery in a subject suffering from a nerve injury. The method can comprise administering a pharmaceutical composition comprising a safe and effective amount of the enzyme, e.g. chondroitinase ABC (ChABC), such as to an injury site of the nerve injury in the subject. The dose may range from about 0.1 U/ml to about 10 U/ml, preferably about 0.5 U/ml to about 5 U/ml, and more preferably about 1 U/ml.

Another aspect of the invention provides (the use of) the enzyme, e.g. ChABC, optionally for preparing a pharmaceutical composition, for promoting axon regeneration and behaviour recovery in a subject. The composition can comprise a safe and effective amount of the ChABC. Such a dose may range from about 0.1 U/ml to about 10 U/ml, preferably about 0.5 U/ml to about 5 U/ml and more preferably about 1U/ml.

One further aspect of the invention provides a pharmaceutical composition suitable for promoting axon re-growth and behaviour recovery, for example in a subject suffering from a nerve injury. It can comprise a safe and effective amount of the enzyme, e.g. chondroitinase ABC (ChABC), for an injury site (e.g. nerve injury) in the subject. Such a dose can range from about 0.1 U/ml to about 10 U/ml, preferably about 0.5 U/ml to about 5 U/ml and more preferably about 1 U/ml.

Additional objects and advantages of the invention will be set forth in part in the description which follows, and in part will be clear from the description, or may be learned by practice of the invention. The objects and advantages of the invention will be realized and attained by means of the elements and combinations particularly pointed out in the appended claims.

It is to be understood that both the foregoing general description and the following detailed description are exemplary and explanatory only and are not restrictive of the invention, as claimed.

### BRIEF DESCRIPTION OF THE DRAWINGS

The foregoing summary, as well as the following detailed description of the invention, will be better understood when read in conjunction with the appended drawings.

In the drawings:

Fig. 1 is a graph illustrating the Basso, Beattie, Bresnahan (BBB) open field locomotor's test scores of the female SD rats after T8 transection in different treatment groups according to the present invention;

Fig. 2A shows the morphologies of spinal cords 8 weeks after T8 transection in different treatment groups according to the present invention;

Fig. 2B shows the histochemical cross sections of spinal cords 8 weeks after T8 transection in different treatment groups according to the present invention;

Fig. 3 shows the histochemical cross sections of the spinal cord injury region 8 weeks after T8 transection in different treatment groups according to the present invention;

Fig. 4 shows the histochemical cross sections of the CS-56 immunostained rat spines in the ChABC 1U/ml group and control group;

Fig. 5 shows the histochemical cross sections of the 2B6 immunostained rat spines in the ChABC 1 U/ml group and the control group;

Fig. 6 shows the histochemical cross section of the HRP traced rat spine in the ChABC 1 U/ml group;

Fig. 7A shows the histochemical cross section of the GAP-43 immunostained rat spine in the ChABC 1 U/ml group;

Fig. 7B shows the high resolution image of the rostral stump lesion site (B) taken from Fig. 7A; and

Fig. 7C is the high resolution image of the central area of the scar tissue (C) taken from Fig. 7A.

### DETAILED DESCRIPTION OF THE INVENTION

The present invention is directed to a method for promoting axon re-growth and behaviour recovery in a subject suffering from a nerve injury. The method can comprise administering a pharmaceutical composition comprising a safe and effective amount of a chondroitinase ABC (ChABC) to an injury site of the nerve injury in the subject. The subject can be a mammal, which can include a human. The nerve injury can include a central nervous system (CNS) injury or peripheral nervous system (PNS) injury, such as spinal cord injury. In one embodiment of the invention, a significantly improved effect on the axon re-growth and a dramatic improvement on behaviour recovery after the ChABC treatment were found.

In accordance with one embodiment of the invention, the subject is administrated by infusing the ChABC to a nerve injury site of the subject, such as through a catheter, e.g. inserted into the spinal cord injury site.

According to one embodiment of the invention, the catheter may be an epidural intrathecal catheter. This can be inserted into the spinal cord injury site and can have one end externalized for infusion of the ChABC. However, the mode of infusing the ChABC shall not be limited as such. Other methods for infusing or administering the ChABC to the subject may also be encompassed by the present invention as long as they provide a non-toxic and effective dosage to the subject. Also, the relative low dose of ChABC to be infused is about 0.5 to about 10 U/ml ChABC, preferably about 0.5 U/ml to about 5 U/ml and more preferably about 1 U/ml. Preferably, the subject may be administered once a week, such as for a human, depending on the individual need of the subject.

The present invention also provides a use of ChABC for preparing a composition for promoting axonal regeneration and behaviour recovery in a subject suffering from a nerve injury. The composition can comprise a relatively low dose of the ChABC. The low dose of ChABC is about 0.5 to about 10 U/ml ChABC, preferably about 0.5 U/ml to about 5 U/ml and more preferably about 1 U/ml. Other active or non-active ingredients may be added without diminishing the therapeutic effect of the ChABC.

It is noted that CSPGs are upregulated via astrocytes and oligodendrocytes to limit axonal regeneration. According to the invention, an infusion of ChABC to the injury site degrades the CSPGs, allowing the axonal regeneration to take place.

In accordance with an example of the invention, an epidural intrathecal catheter is inserted via a wound to a spinal cord injury site of the subject suffering from a nerve injury. The wound may then be closed, leaving one end of the epidural intrathecal catheter externalized for infusion of the ChABC. A significant effect of ChABC on the axon re-growth as well as behaviour recovery of a subject suffering from a nerve injury was expectedly found in the invention.

The ChABC or the composition containing ChABC may be given depending on the individual subject's need, such as once a week in a human. In one embodiment of the invention, the subject (such as a rat) was administrated with 1 U/ml of ChABC every other day for two weeks (total 8 times).

The invention will now be described in further detail with reference to the following specific, non-limiting examples.

**EXAMPLES**

### Example 1: The insertion of catheter and infusion of chondroitinase ABC after T8 complete spinal cord transection

Female Sprague-Dawley (SD) rats (each weighed 250 to 300g) were used in the experiment. And all surgery was done under inhalation anesthesia with isoflurance and above homoeothermic blanket for keeping body temperature at about 37°C. The skin over foramen magnum was prepared and a longitudinal midline incision was made for exposure the for magnum and C1 lamina. The epidural space was created just above the C1 lamina and the catheter was inserted to reach the T8-T9 level. The wound was closed layer by layer and the catheter was externalized for infusion of chondroitinase ABC.

One week after catheter insertion and the confirmation of normal hindlimbs movement, these animals received T8 total laminectomy for exposure the spinal cord and the inserted catheter at T8 level. The complete transection of spinal cord was done under the manner of lifling up of both stumps for the completion of spinal cord transection and the both stumps were approximated together. The catheter was cut at the T8 cord level also for the infusion of ChABC. The first dose of ChABC was given 2, 4 and 6 weeks after the transection of spinal cord through the epidural intrathecal catheter. The wound was closed layer by layer. After surgery, the animals were immediately placed on homoeothermic blanket and kept under heating lamp for first week. Urine was discharged via catheterization twice daily until the urination function became normal. To prevent urinary tract infection, prophylactic antibiotic was administered once daily until the animal voided its own urine.

The female SD rats were divided into three groups according to the interval between spinal cord transection and infusion of ChABC. The enzyme was infused at three different concentrations, 1, 5 and 10 U/ml, after complete spinal cord transection. ChABC was given every other day for a duration of two weeks (total 8 times, each time 6 µl) under inhalation anesthesia. The externalized tube was cut and removed two weeks after the last infusion of ChABC. In control group of the female SD rats received (1) T8 spinal cord transection only (T8 tx only), (2) T8 spinal cord transection and insertion of epidural intrathecal catheter only (T8 tx + tube only), (3) injury, catheter insertion and normal saline infusion only (T8 tx + saline) until 8 weeks after the final dose of ChABC infusion.

Example 2: Behavioural assessment

All animals received behavioural test every week post-surgery for 8 weeks. All behavioural tests were videotaped and two examiners who participated in behaviour evaluation were blinded to each group. The hindlimb locomotor's behaviour of rats was evaluated by the Basso, Beattie, Bresnahan (BBB) open field locomotor's test. Each session lasted 5 minutes. The open field locomotor's activity score ranging from 0 to 21 (0 for no movement, and 21 for normal movement) was determined by observation and scoring of behaviours involving the trunk, tail and hindlimb.

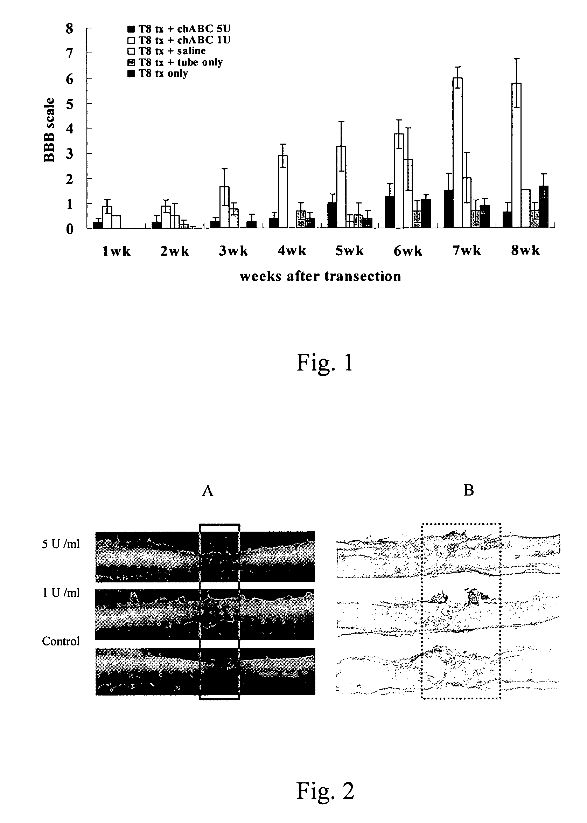

Referring to Fig. 1, there were statistical differences in the hind limbs locomotor's function evaluation between ChABC 1U/ml group (T8 tx + chABC 1 U), ChABC 5U/ml group (T8 tx + chABC 5U) and the control groups (T8 tx + saline, T8 tx + tube only and T8 tx only) 3 weeks after surgery and ChABC treatment, and the differences persisted up to 8 weeks later.

Example 3: Anterograde labeling of axons crossing the transection site

Following 8 weeks, the female SD rats were anesthetized under inhalation of isoflurane. The spinal cord was exposed over the T10 level and 4% WGA-HRP was injected into the motor cortex of the brain via a micro-syringe. Three sites (to disperse injecting on each side 0.24 µl × 3) were injected by a slow pumping system. The animals were sacrificed for transcardial perfusion with 4 % paraformaldehyde under anesthesia two days after microinjection. The spinal cord, together with brain stem were removed, post-fixed and cryo-preserved in 30 % sucrose overnight for serial section. The spinal cord was sectioned longitudinally and the brain stem was sectioned coronary at 30 µm thickness.

Example 4: Immunohistochemistry

The spinal cords were collected, immersed in 4 % paraformaldehyde in phosphate buffer overnight and then transferred to a 30 % sucrose solution. The horizontal or transverse cry-sections (each having a 20 um thickness) of the spinal cords were placed on poly-L-lysine-coated slide for immunostaining. Following incubation with 5 % bovine serum albumin in PBS for 30 min, monoclonal antibodies to CS-56 (1:500; Sigma, St. Louis, MO), 2B6 (1:5000; Seikagaku Corporation), GAP-43 (1:1000; Sigma, St. Louis, MO) and NG2 were used for primary antibodies. Appropriate secondary antibody plus avidin biotin complex (ABC) procedure was used for peroxidase staining. Negative control was verified by omission of the primary antibody to CS-56 (a type of CSPG), 2B6 (degradation products of CSPG) and GAP-43 (a marker for newly formed axon), antibodies were also used for the evaluation of the degradation of CSPG and the regeneration of axons across the lesion site.

The morphological scar tissues were observed in the ChABC 1U/ml group and ChABC 5U/ml group by comparison with the control group as shown in Fig 2A. However, large cysts were observed near the transection site in the ChABC 5U/ml group as shown in Fig 2B.

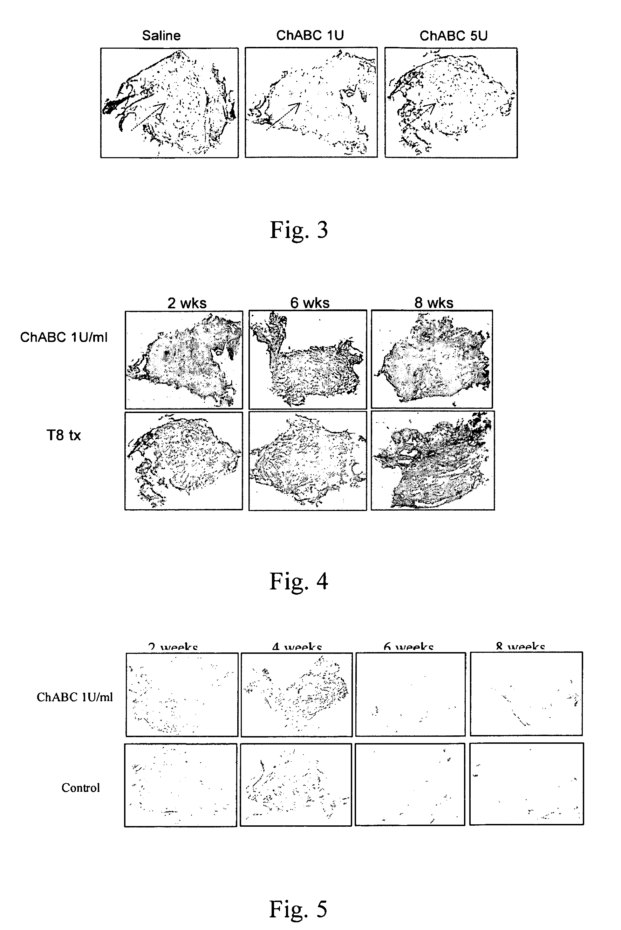

The immunostaining of CS-56, which was a complete CSPGs structure of scar over the region of spinal cord injury revealed dramatic decrease in the group treated with ChABC 1 U/ml compared with the ChABC 5 U/ml group and control groups 8 weeks after spinal cord injury and ChABC treatment as shown in Fig. 3. The undigested scar tissues were strong immunopositive of CS-56. The lowest expression of CS-56 within 2 weeks after 1 U/ml ChABC treatment was detected, but CS-56 still increased for 4 weeks after ChABC treatment as shown in Fig 4. In ChABC 1U/ml group, the CS-56 immunostaining was reached the peak level 2 weeks after spinal cord injury compared with the T8-transection group whose CS-56 immunostaining reached peak level at 6 weeks and persisted up to 8 weeks.

The 2B6 expression in the ChABC 1U/ml group was stronger than the control groups. There were no differences in the immunostaining of 2B6 (for degradation products of CSPGs by ChABC) between the ChABC 1 U/ml group, ChABC 5 U/ml group and control group for 8 weeks after ChABC treatment. Referring to Fig. 5, the 2B6 immunostaining was observed in the 1U/ml ChABC group for 2 weeks after ChABC treatment to compare with the control group.

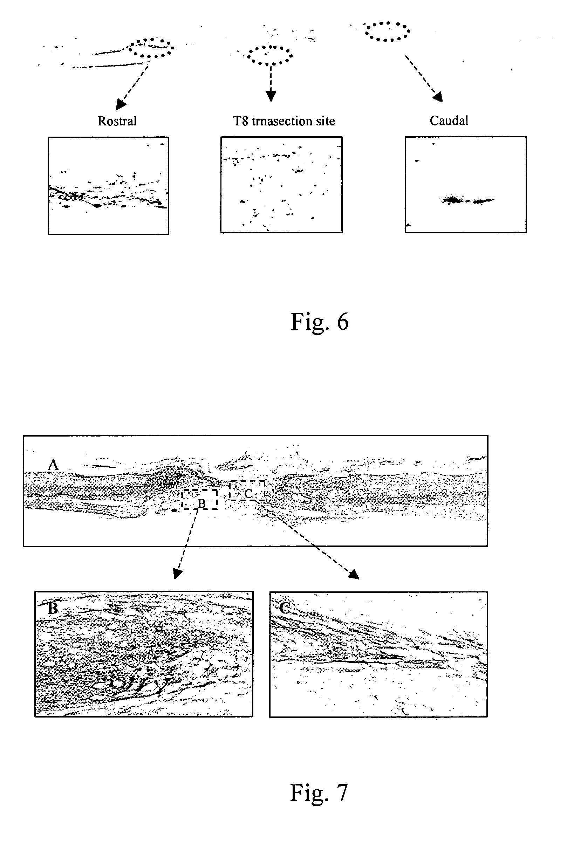

Wheat-germ agglutinated horse radish peroxidase (WGA-HRP) labeled axons were observed across the transection site in the ChABC 1 U/ml group as shown in Fig 6. Small size scar did not block the re-growth of axons. Referring to Fig. 7A, a few GAP-43 immunopositive processes were observed across the T8 transection site in ChABC 1U/ml group 8 weeks after transection. In the rostral stump lesion site, a large number of axons were observed as shown in Fig. 7B. In the central area of the scar tissue, less and thinner axons were found as shown in Fig. 7C.

It should be understood by one having an ordinary level of skill in the art in view of the present disclosure that the present method is equivalently applicable to treating any vertebrate suffering from the nerve injury. The vertebrates include but not limited to humans, commercially relevant mammals such as cattle, pigs, horses, sheep, cats, dogs, mice, rats, rabbits, and pets, birds including commercially relevant birds such as chickens, ducks, geese, and turkeys.

It will be appreciated by those skilled in the art that changes could be made to the embodiments described above without departing from the broad inventive concept thereof. It is understood, therefore, that this invention is not limited to the particular embodiments or Examples disclosed, but covers modifications within the present invention as defined by the appended claims.

## Claims

1. The use of chondroitinase ABC (ChABC), optionally for the manufacture of a medicament or pharmaceutical composition, for:
(a) promoting axon regeneration and/or behaviour recovery;
(b) promoting axon re-growth and/or behaviour recovery; and/or
(c) treating a nerve injury.

2. The use according to claim 1, wherein the pharmaceutical composition comprises from 0.1 U/ml to 10 U/ml of the ChABC or from 0.5 U/ml to 5 U/ml of the ChABC.

3. The use according to claim 1 or 2, wherein the pharmaceutical composition comprises about 1 U/ml of the ChABC.

4. The use according to any preceding claim, wherein the pharmaceutical composition is administered to the injury site by infusion, for example via an epidural intrathecal catheter.

5. The use according to any preceding claim, wherein from 1 µl to about 100 µl of the pharmaceutical composition is administered to the injury site per infusion and/or an infusion is performed once daily or once every other day.

6. The use according to any preceding claim, wherein the nerve injury comprises a central nervous system (CNS) injury or a peripheral nervous system (PNS) injury.

7. The use according to any preceding claim, wherein the nerve injury comprises a spinal cord injury and/or the use is for vertebrate, such as a mammal (e.g. a human).

8. A pharmaceutical composition suitable for promoting axon re-growth or regeneration and/or behaviour recovery or for treating a nerve injury, the pharmaceutical composition comprising a safe and effective amount of chondroitinase ABC (ChABC), and a pharmaceutically acceptable carrier.

9. A pharmaceutical composition comprising from 0.1U/ml to about 10U/ml of chondroitinase ABC (ChABC).

10. A pharmaceutical composition according to claim 8 or 9 comprising from 0.5 U/ml to 5 U/ml of ChABC, such as about 1 U/ml of ChABC.
